# EUROPEAN PATENT APPLICATION

(11) **EP 4 130 030 A1**
(43) Date of publication of application: **08.02.2023**
(21) Application number: 21781075.3
(22) Date of filing: 29.03.2021
(51) Int. Cl.: C07K 14/745, G01N 33/53, G01N 33/543, G01N 33/92

(54) **QUANTIFICATION METHOD FOR MODIFIED HDL, AND ANALYSIS REAGENT FOR USE IN SAME METHOD**

(30) Priority: 30.03.2020 JP 2020059965
(71) Applicant: SHINSHU UNIVERSITY, Matsumoto City, Nagano, 390-8621 (JP)
(72) Inventor: SAWAMURA, Tatsuya, Matsumoto City, Nagano 3908621 (JP); KAKINO, Akemi, Matsumoto City, Nagano 3908621 (JP)
(74) Representative: Richly & Ritschel Patentanwälte PartG mbB
(86) International application number: PCT/JP2021/013229
(87) International publication number: WO 2021/200797

(57) **Abstract**

The present invention provides a method for quantifying modified HDL contained in a sample, comprising binding a modified HDL to a modified HDL-binding protein, and quantifying a complex of the modified HDL-binding protein and the modified HDL, wherein the modified HDL-binding protein comprises a protein having a sequence of an L chain region of Factor V, of a variant thereof.

## Description

### [Technical Field]

The present invention relates to a method for quantifying modified HDL that can be used to determine the risk of lifestyle-related diseases and its healing effect, and an analytical reagent used therefor.

### [Background Art]

HDL (High-Density Lipoprotein) is a kind of so-called cholesterol, and has a high specific gravity and a small particle size. It is known that HDL is responsible for the transport of cholesterol from tissues to the liver. HDL is sometimes referred to as "good cholesterol" because high blood levels reduce the risk of arteriosclerosis. On the other hand, LDL (Low-Density Lipoprotein) is known as "bad cholesterol." The concentrations of HDL and LDL are used as indicators for health examination.

As for the physiological activities of HDL, it not only suppresses the oxidation of LDL, but also reduces cytotoxicity due to the oxidized LDL through an increase in NO, and the like, thereby exerting the antiarteriosclerotic effect. However, it has been clarified that patients with coronary artery disease have an increased modified HDL in their blood, which acts on the blood vessels via LOX-1 to promote arteriosclerosis, and that a large amount of modified ApoAI, which is an apoprotein of HDL, accumulates in human arteriosclerotic lesions. These results show that, in order to use HDL as an indicator for health examination, it is necessary not only to measure the concentration of HDL, but also to separately quantify the HDL that has its original physiological activity and the HDL whose activity has changed due to modification or the like. In particular, if it is possible to quantify the modified HDL, it is believed that it can be used to verify diseases related to the circulatory system and blood, including the aforementioned coronary artery diseases, and the risk thereof.

Patent Document 1 discloses a method for measuring dysfunctional HDL contained in a sample, including contacting the sample with a receptor for dysfunctional HDL to bind the dysfunctional HDL in the sample to the receptor, and detecting the dysfunctional HDL bound to the receptor, wherein the receptor is LOX-1 or a variant thereof, the receptor is immobilized on a carrier, and an antibody that recognizes the dysfunctional HDL is further bound to the receptor-bound dysfunctional HDL. This technique aims to provide a technique for measuring dysfunctional HDL that can widely detect dysfunctional HDL in various molecular forms, and to provide a technique for detecting lifestyle-related diseases using this technique.

In addition, in Patent Document 2, the present inventors disclosed a fusion protein in which an entire protein sequence or a partial fragment protein sequence of ApoA1 is linked directly or via a spacer to an LOX-1 binding protein sequence that binds to LOX-1, and a high-density lipoprotein measurement kit using the same. By this technique, HDL that has undergone various modifications can be measured by measuring HDL that is simultaneously recognized by LOX-1 and an anti-ApoA1 antibody. In addition, this technique uses a fusion protein in which a protein that specifically binds to LOX-1 is fused with ApoA1. The protein that specifically binds to LOX-1 has, for example, an LOX-1 binding site of an antibody against LOX-1 (anti-LOX-1 antibody). The aim is to obtain a fusion protein that can simultaneously recognize HDLs that have undergone various modifications, that can be used to measure the physiological activity itself by measuring the receptor binding activity, that can be stored for a long time without containing lipids, that can be adjusted reproducibly, and that can serve as a reference that can reduce variations between the measurement tests and increase the reliability.

### [Citation List]

### [Patent Documents]

[Patent Document 1] Japanese Patent Granted Publication No. 6231307
[Patent Document 2] Japanese Patent Application No. 2018-024007

### [Summary of Invention]

### [Technical Problem]

Theoretically, modified HDL can be quantified in the laboratory by the technique described in Patent Document 1. The technique of Patent Document 2 of the present inventors is more sophisticated than the conventional technique. By applying these techniques, it was expected that it would be easy to establish a method that can be used to quantify modified HDL, even if it is not very sophisticated.

However, the current situation is that the technique of Patent Document 1 is not in widespread use. Furthermore, even with the technique of Patent Document 2 of the present inventors, the results of quantification of modified HDL were sometimes inconsistent when performed under non-laboratory conditions and when using different specimens. These differences are considered to be due to various factors, such as differences in laboratory conditions and testing techniques of those who perform the techniques.

Considering the above-mentioned historical background and the fact that measurement should be possible even under somewhat poor conditions for practical clinical application, the present inventors have performed intensive studies with the goal of improving the quantification technique for modified HDL to a more versatile quantification technique.

The present invention has been made in view of the circumstances as described above, and an object of the present invention is to provide a method for quantifying modified HDL that can quantify modified HDL more stably and accurately than before, and is useful for determining cardiovascular diseases, diabetes, diabetic diseases, etc., or progression thereof, and an analytical reagent used for the quantification method.

### [Solution to Problem]

In order to solve the above problems, the present invention includes the following aspects.
[1] A method for quantifying modified HDL contained in a sample, including binding a modified HDL to a modified HDL-binding protein, and quantifying a complex of the modified HDL-binding protein and the modified HDL, wherein the modified HDL-binding protein includes a protein having a sequence of an L chain region of Factor V, or a variant thereof.
[2] The method for quantifying modified HDL as described above, wherein the modified HDL-binding protein includes a sequence of a phospholipid recognition site of Factor V.
[3] The method for quantifying modified HDL as described above, wherein the modified HDL-binding protein includes a sequence of Factor V.
[4] The method for quantifying modified HDL as described above, wherein the modified HDL-binding protein is a protein having a site partially homologous to a C1-C2 domain, which is a phospholipid recognition site of Factor V.
[5] The method for quantifying modified HDL as described above, wherein the protein having a site partially homologous to a C1-C2 domain is MFG-E8, Del-1 of Factor VIII.
[6] The method for quantifying modified HDL as described above, wherein the modified HDL-binding protein is a recombinant protein.
[7] The method for quantifying modified HDL as described above, wherein the quantification is performed by forming a complex of the modified HDL and the modified HDL-binding protein, and quantifying the complex by immunoassay.
[8] The method for quantifying modified HDL as described above, wherein the quantification by immunoassay is performed by an ELISA method using an ELISA plate on which the modified HDL-binding protein or a protein that binds to the modified HDL-binding protein is immobilized.
[9] The method for quantifying modified HDL as described above, wherein the quantification is performed by forming a complex of the modified HDL, the modified HDL-binding protein and an LOX-1 protein, and quantifying the complex by immunoassay.
[10] The method for quantifying modified HDL as described above, wherein serum or plasma is used as a sample in the quantification.
[11] The method for quantifying modified HDL as described above, further including adding the modified HDL-binding protein to a sample, wherein serum is used as a sample in the quantification.
[12] An analytical reagent used for quantifying modified HDL for determining cardiovascular disease, diabetes or diabetic disease, the risk thereof, or the degree of progression thereof, comprising a modified HDL-binding protein capable of binding to a modified HDL, wherein the modified HDL-binding protein comprises a protein having a sequence of an L chain region of Factor V, or a variant thereof.
[13] The analytical reagent as described above, wherein the modified HDL-binding protein comprises a sequence of a phospholipid recognition site of Factor V.
[14] The analytical reagent as described above, wherein the modified HDL-binding protein comprises a sequence of Factor V.
[15] The analytical reagent as described above, wherein the modified HDL-binding protein is a protein having a site partially homologous to a C1-C2 domain, which is a phospholipid recognition site of Factor V.
[16] The analytical reagent as described above, wherein the protein having a site partially homologous to a C1-C2 domain is MFG-E8, Del-1 or Factor VIII.
[17] The analytical reagent as described above, wherein the modified HDL-binding protein is a recombinant protein.
[18] The analytical reagent as described above, wherein the cardiovascular disease is arteriosclerosis, or myocardial infarction or stroke caused by a progression of arteriosclerosis.

### [Advantageous Effects of Invention]

According to the present invention, a method for quantifying modified HDL that can quantify modified HDL more stably and accurately than before, and is useful for determining cardiovascular diseases, diabetes, diabetic diseases, etc., or the degree of progression thereof; and an analytical reagent for use in the quantification method can be obtained.

### [Brief Description of Drawings]

FIG. 1 is a schematic diagram showing the method for quantifying modified HDL according to the first embodiment.
FIG. 2 is a schematic diagram showing the method for quantifying modified HDL according to the second embodiment.
FIG. 3 is a graph showing the detection sensitivity of modified HDL in Test Example 1.
FIG. 4 is a graph showing the detection sensitivity of modified HDL in plasma in Test Example 2.
FIG. 5 is a graph showing the detection sensitivity of modified HDL in serum in Test Example 2.
FIG. 6 is a graph showing the detection sensitivity of modified HDL in plasma at different concentrations of Factor V in Test Example 2.
FIG. 7 is a graph showing the detection sensitivity of modified HDL in serum at different concentrations of Factor V in Test Example 2.
FIG. 8 is a graph showing the immobilized amount of Factor V in Test Example 4.
FIG. 9 is a graph showing the binding test of modified HDL in Test Example 4.
FIG. 10 is a graph showing the quantification of modified HDL in plasma and serum in Test Example 5.

### [Description of Embodiments]

The method for quantifying modified HDL and the analytical reagent according to the present invention will be described below with reference to embodiments. However, the present invention is not limited to the following embodiments.

### (First embodiment)

### (Method for quantifying modified HDL)

In the method for quantifying modified HDL of the present embodiment, a modified HDL and a modified HDL-binding protein are allowed to bind to quantify the modified HDL contained in a sample, and the complex of the modified HDL-binding protein and the modified HDL is quantified.

Examples of the modified HDL in the present embodiment include an HDL having a structural difference from the original HDL that has physiological activity in vivo, and particularly refers to an HDL whose physiological activity is reduced due to the structural differences. Examples of the mechanisms by which an HDL becomes a modified HDL include factors such as chemical modification and partial or complete degradation of the constituent proteins, genetic defects and mutations, abnormalities or changes in higher-order structure, and binding with molecules that eliminate activity.

Examples of such modified HDL include, an oxidized HDL in which any site of the protein or the lipid constituting HDL is oxidized. Examples of such modified HDL include HClO-HDL, HNE-HDL, carbamylated HDL and MDA-HDL. These modified HDLs are known to increase the risk of developing various diseases such as arteriosclerotic diseases such as heart disease and ischemic disease that damages vascular endothelial cells in the vascular wall and causes arteriosclerosis and thrombus formation.

In the method for quantifying modified HDL of the present embodiment, a modified HDL and a modified HDL-binding protein are allowed to bind, and a complex of the modified HDL-binding protein and the modified HDL is quantified.

The modified HDL-binding protein of the present embodiment broadly refers to a protein that can directly interact with a modified HDL, for example, a protein that itself can bind to a modified HDL.

A modified HDL may acquire an activity of interacting with receptors that cause adverse reactions in the body. In this case, the modified HDL binds to the receptor via a modified HDL-binding protein, that is, the modified HDL binds directly to the modified HDL-binding protein, and the modified HDL-binding protein binds to the receptor.

Examples of the modified HDL-binding protein include Factor V, variants thereof, and partially homologous proteins thereof. Examples of the receptor that may bind to the modified HDL via another protein include an LOX-1 protein described later.

In the present embodiment, the modified HDL-binding protein includes a protein having a sequence of the L chain region of Factor V, of a variant thereof.

Factor V is known as a protein that constitutes the coagulation system, and is a protein of approximately 330 kDa composed of an A1-A2-B-A3-C1-C2 domain, and it is roughly divided into an H chain (heavy chain) composed of an A1-A2 domain and an L chain (light chain) composed of an A3-C1-C2 domain. It is known that the H chain mainly binds to cell membranes, and the L chain mainly recognizes and binds to phospholipids via the C1-C2 domain.

A mutant refers to one in which a part of a sequence has been replaced, and in the present embodiment, it is preferable that a site other than the L chain region of Factor V be mainly replaced.

Since the L chain region of Factor V is a site that directly binds to the modified HDL, the modified HDL-binding protein is a protein containing this site or a variant thereof, and therefore, the modified HDL and the modified HDL-binding protein can be bound and used for quantification.

In addition, by using a protein having a sequence of the L chain region that is a part of Factor V as the modified HDL-binding protein, it is possible to use a protein having a structure different from Factor V while having a site that binds to the modified HDL. That is, a protein that is more readily available than Factor V or a protein that is easy to perform a test can be used. As the protein that is more readily available than Factor V, for example, a protein that has a full length shorter than Factor V and is easy to produce a recombinant protein can be used by using only the L chain region and minimum sequence. As the protein that is easy to perform a test, a protein that can be easily stored or immobilized on a plate can be used by changing the sequence other than the L chain.

It is also preferred that the modified HDL-binding protein include a sequence of a phospholipid recognition site of Factor V. Among the sequences of Factor V, the phospholipid recognition site included in the L chain can recognize and effectively bind to the modified HDL.

Preferably, the modified HDL-binding protein includes a sequence of Factor V. Here, the sequence of Factor V has the full length of Factor V protein, and Factor V itself may be used, or a protein fused or bound with other proteins, a modified protein or the like may be used.

The modified HDL-binding protein is also preferably a protein having a site partially homologous to the C1-C2 domain, which is the phospholipid recognition site of Factor V. Examples of the protein having a site partially homologous to such C1-C2 domains include MFG-E8, Del-1 and Factor VIII. Also, these proteins, mutants thereof, and proteins partially including the sequences of these proteins may be used.

Modified HDL-binding protein is also preferably a recombinant protein, for example. Here, the recombinant protein is a protein obtained by genetic recombinant expression. The recombinant protein may be fused with another structure or mutated as necessary.

In the present embodiment, the modified HDL contained in a sample is quantified. Here, the sample broadly refers to a sample containing a component extracted from living organisms. It is preferable to use a liquid form of the above component or add a liquid to the above component to obtain a liquid sample. As the sample, for example, body fluids of organisms can be directly used. For example, human blood of saliva can be used as the body fluids of organisms. Serum, plasma, or the like can be used as the blood sample. In the present embodiment, these body fluids can be used as samples and subjected to the above-described quantification.

In the present embodiment, serum is preferably used as a sample. In the present embodiment, a modified HDL-binding protein having a sequence of the L chain region of Factor V that directly binds to a modified HDL is allowed to bind to a modified HDL in a sample. Therefore, the modified HDL can be stably and accurately quantified even when the serum, which may not contain a factor that directly binds to the modified HDL, can be used as a sample. In addition, serum is easier to obtain and store than other body fluid samples such as plasma. In fact, as blood samples, specimens that are cryopreserved in the form of serum are common internationally, and this is a useful technique.

The present inventors paid attention to the fact that in the conventional technique that uses a protein that interacts with the modified HDL such as LOX-1 to quantify modified HDL, although it is possible to detect the modified HDL when plasma is used as a sample, the modified HDL is rarely detected and cannot be quantified accurately when serum is used as a sample. Therefore, the present inventors predicted that the measurement using serum as a sample would be possible by supplementing the measurement system with substances that are decomposed and lost in the process of forming serum from blood or plasma.

The present inventors focused on Factor V as the supplementary substance. When the modified HDL binds to LOX-1 in an in vivo reaction, it binds via Factor V. Specifically, the phosphatidylserine (PS) recognition site of Factor V binds to the modified, and the LOX-1 recognition site binds to LOX-1.

As will be described later, when serum is used as a sample and Factor V is added to the sample, the detection sensitivity of modified HDL in serum increases substantially according to the added amount. Therefore, by adding a modified HDL-binding protein such as Factor V to a sample such as serum, the modified HDL can be quantified.

Known means for quantification of protein (measurement of protein amount) can be used for the above quantification. For example, immunological techniques can be used for the above quantification.

Enzymes (enzyme immunoassay, ETA, ELISA), fluorescent substances (fluorescence immunoassay, FIA), radioactive substances (radioimmunoassay, RIA), etc. can be appropriately used for the quantification and immunoassay using immunological techniques. Among these, ELISA is preferable because it is relatively inexpensive, simple, and can analyze a large number of samples.

For example, in the present embodiment, ELISA method can be used for the quantification. Specifically, a modified HDL-binding protein is immobilized on an ELISA plate. Known methods can be appropriately used for the immobilization of the modified HDL-binding protein. For example, a receptor can be bound to a known carrier (support).

A sample is added to the ELISA plate, the modified HDL contained in the sample is allowed to bind to the modified HDL-binding protein, the ELISA plate is washed to remove the unbound substances, and an antibody capable of detecting the modified HDL or the complex is used, thereby making it possible to quantify the complex of the modified HDL and the modified HDL-binding protein in the target to be quantified.

As the antibody that binds to the modified HDL or the complex, an antibody that binds to the sequence of HDL can be appropriately used. In the present embodiment, since the complex of the modified HDL and the modified HDL-binding protein on the ELISA plate is detected, the substance detected on the plate is a modified HDL. In the present embodiment, both monoclonal and polyclonal antibodies can be used as the antibody that binds to the modified HDL.

More specifically, in the present embodiment, a modified HDL and a modified HDL-binding protein are bound to form a complex, and this complex can be quantified by immunoassay.

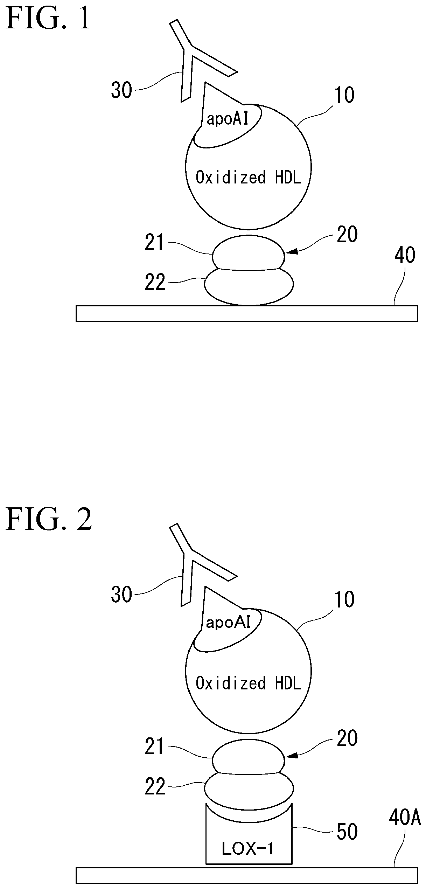

This immunoassay will be described with reference to a schematic diagram in FIG. 1. For example, a modified HDL-binding protein 20 is immobilized on a plate 40. In the present embodiment, the modified HDL-binding protein 20 is, for example, a protein having an L chain region 21 and an H chain region 22, and having homology to Factor V protein. The plate 40 is an ELISA plate. When immobilization is performed with a known carrier, a structure in which the H chain region 22 is immobilized on the plate 40 is formed on the plate 40. The plate 40 is then washed to remove the unbound modified HDL-binding protein 20.

A sample derived from a body fluid is injected into the plate 40. A modified HDL 10 in the sample binds to the L chain region 21 on the plate 40. The complex on the plate 40 is quantified with an antibody 30 that binds to the modified HDL or the complex. In the present embodiment, the antibody 30 is an anti-apolipoprotein AI antibody (anti-ApoA1).

In the present embodiment, since the modified HDL-binding protein that directly binds to the modified HDL is immobilized on the plate without using a conventional modified HDL receptor such as LOX-1 or the like, the modified HDL receptor is unnecessary. For example, as the modified HDL-binding protein, if an easily available protein such as a protein with a full length shorter than Factor V as described above is used, quantitation can be performed more easily than using a modified HDL receptor such as LOX-1 for quantification.

### (Analytical reagent used for quantification of modified HDL)

Next, the analytical reagent used in the above-described method for quantifying modified HDL in body fluid will be described.

This analytical reagent can be used to quantify the modified HDL in body fluid, thereby quantifying the modified HDL in body fluid to determine cardiovascular diseases, diabetes, or diabetic diseases, or the degree of progression thereof.

The analytical reagent contains the modified HDL-binding protein described above. By containing the modified HDL-binding protein, a modified HDL having physiological activity can be quantified.

### (Other configurations)

The analytical reagent of the present embodiment may contain other components that may be contained in the immunological analysis reagent.

Moreover, the analytical reagent of the present embodiment may be provided as a kit including a plurality of the above-described configurations. For example, the kit may include an analytical reagent containing the modified HDL-binding protein for the quantitation of the modified HDL. The kit may also contain a carrier, an ELISA plate, a chromogenic substrate, an antibody, or the like for use in the above-described quantification procedure.

It may also contain an ELISA plate on which the above-described proteins are immobilized. For example, an analytical plate used for quantifying modified HDL to determine cardiovascular diseases, diabetes or diabetic diseases, the risk thereof, or the degree of progression thereof, wherein an ELISA plate on which a modified HDL-binding protein capable of binding to a modified HDL is immobilized, and the modified HDL-binding protein contains a protein having a sequence of the L chain region of Factor V, or a variant thereof, can also be provided.

### (Effect of the present embodiment)

The method for quantifying modified HDL of the present embodiment can stably and accurately quantify a modified HDL as compared to conventional techniques. The term "stably and accurately quantify" of the present embodiment means that the method of the present embodiment can quantify a modified HDL with a constant high sensitivity regardless of sample conditions, such as contents other than the modified HDL, while the conventional method has a difference in the detection sensitivity itself depending on the samples or the detection sensitivity is low.

The method for quantifying modified HDL of the present embodiment can stably and accurately quantify a modified HDL, and can be used to determine the diseases, the risk thereof, and the progress thereof. As the diseases, diseases that are considered to be related to HDL are widely targeted, and examples thereof include cardiovascular diseases, diabetes, and diabetic diseases. Examples of cardiovascular diseases include, in particular, myocardial infarction or stroke caused by arteriosclerosis of its progression.

### (Application of the present embodiment)

The method for quantifying modified HDL and the analytical reagent of the present embodiment can be used, for example, in a method for diagnosing the above-described cardiovascular diseases and/or diabetes or diabetic diseases by measuring modified HDL in the quantification of HDL in body fluid.

In the diagnostic method, for example, it is also possible to diagnose the risk and/or the degree of progression of the diseases from the change in the ratio of the amount of modified HDL with respect to the total amount of HDL in the measurement of modified HDL in the sample.

This diagnostic method also includes a process of preparing a calibration curve, comparison table of the like using a protein standard, healthy body fluid samples or the like as a standard, and diagnosing by comparing the result of the quantification of the specimen sample to be diagnosed with the calibration curve, comparison table or the like.

In addition, it can also be used for determining and diagnosing obesity-related diseases such as hypertension or sleep apnea syndrome, the risk thereof, and/or the degree of progression thereof.

### (Second embodiment)

In the second embodiment, a complex of a modified HDL, a modified HDL-binding protein and an LOX-1 protein is formed and this complex is quantified by immunoassay.

The same reference signs are used in the drawings for the same configurations as in the first embodiment, and the description thereof will be omitted.

In other words, in contrast to the conventional techniques that attempt to quantify by detecting a complex of a modified HDL and an LOX-1 protein, the present embodiment attempts to detect more stably and reliably by additionally interposing a modified HDL-binding protein. In the present embodiment, the techniques and equipment used in the conventional technique using an LOX-1 protein have been improved to perform the quantification.

LOX-1 is a molecule discovered by the present inventors (Sawamura T et al. Nature 386, 73-77, 1997) and is known as a lectin-like oxidized LDL receptor. The detailed structure of LOX-1 has been clarified that LOX-1 is a single-pass membrane protein and has a lectin-like domain. It has been known that this lectin-like domain is a recognition site of oxidized LDL (Japanese Unexamined Patent Application, First Publication No. H9-98787, etc.), an soluble components are also present in blood, and the modified high-density lipoprotein (HDL) acts as a ligand for LOX-1 (Japanese Unexamined Patent Application, First Publication No. 2012-100585, Japanese Patent Granted Publication No. 6231307, etc.).

For the LOX-1 protein, it is also preferable to use, for example, a recombinant LOX-1 protein. Here, the recombinant LOX-1 protein refers to an LOX-1 protein obtained by genetic recombinant expression. The recombinant LOX-1 protein may be fused with other structures, or mutated, if necessary.

In the immunoassay described above, the quantification of the complex of the modified HDL, the modified HDL-binding protein and the LOX-1 protein can be performed using a so-called sandwich ELISA.

The outline of the quantification method of the present embodiment is shown in FIG. 2. In this quantification method, for example, an LOX-1 protein 50 is immobilized on a plate 40A. A modified HDL-binding protein 20 is allowed to bind to the LOX-1 protein 50 on this plate 40A. By injecting a body fluid-derived sample on this plate 40A, a complex of the LOX-1 protein 50 and the modified HDL-binding protein 20 is allowed to bind to a modified HDL10. The complex on the plate 40A can then be quantified with an antibody 30 that binds to the above-described modified HDL or the complex. An anti-Factor V antibody can also be used as the antibody 30.

The present embodiment may include a step of adding the modified HDL-binding protein to a sample, in which serum is used as a sample. By adding this sample to the above-described ELISA plate on which LOX-1 is immobilized, a complex in which the modified HDL-binding protein and the modified HDL are bound to the LOX-1 on the ELISA plate can be formed, thereby making it possible to quantify the complex on the ELISA plate with an antibody that binds to the modified HDL or the complex.

The analytical reagent used in the quantification method of the present embodiment may contain a recombinant LOX-1 protein for the quantification of the modified HDL having pathological activity. As described above, since the recombinant LOX-1 protein interacts with the modified HDL, this analytical reagent makes it possible to quantify the modified HDL by immunoassaying the complex of the modified HDL, the modified HDL-binding protein and LOX-1.

Moreover, the analytical reagent of the present embodiment may be provided as a kit including a plurality of the above-described configurations. For example, the kit may include an analytical reagent containing the modified HDL-binding protein for the quantitation of the modified HDL and an analytical reagent containing the LOX-1 protein.

### [EXAMPLES]

Examples are shown below. In addition, the present invention is not limited to the Examples.

### (Test Example 1)

### (Verification of detection sensitivity of modified HDL by Factor V)

First, the effect of Factor V on the binding between a modified HDL and LOX-1 was examined, LOX-1 being a receptor for the modified HDL.

A plasma or serum sample containing a modified HDL was added to a plate on which LOX-1 was immobilized, and a test was performed in which a complex of LOX-1 and the modified HDL was detected with an antibody of the modified HDL (anti-apoA1 antibody). At this time, if the detection sensitivity was improved depending on the addition amount of Factor V and stable detection sensitivity was obtained, the binding mechanism of Factor V and a modified HDL can be used for the quantification of modified HDL.

The ELISA method was performed according to the following procedures.

0.15 µg/well) of a recombinant human LOX-1 (61-273aa) was immobilized on a 384-well plate. After washing with PBS twice, blocking was performed with 1% casein-Na. After washing with PBS three times, Factor V-added plasma or serum samples were incubated for 2 hours at room temperature. A sample diluent (1% casein-Na, 10 mM HEPES, 150 mM NaCl, pH 7.4, 100 µM APMSF) was used. After washing with PBS three times, an anti-apoA1 antibody (chicken #1) adjusted to 1 µg/ml was added and incubated at room temperature for 1 hour. After washing with PBS three times, a 4,000-fold diluted HRP-labeled anti-chicken IgY antibody was added and incubated at room temperature for 1 hour. After the antibody reaction, the plate was washed with PBS five times, a TMB solution (manufactured by Bio-Rad) was added to the plate, and the reaction was allowed to proceed at room temperature. The reaction was stopped with 2M sulfuric acid and the absorbance was measured at 450 nm. The concentration of the modified HDL (ng/ml) was calculated using a calibration curve prepared using an oxidized HDL oxidized with copper sulfate as a standard.

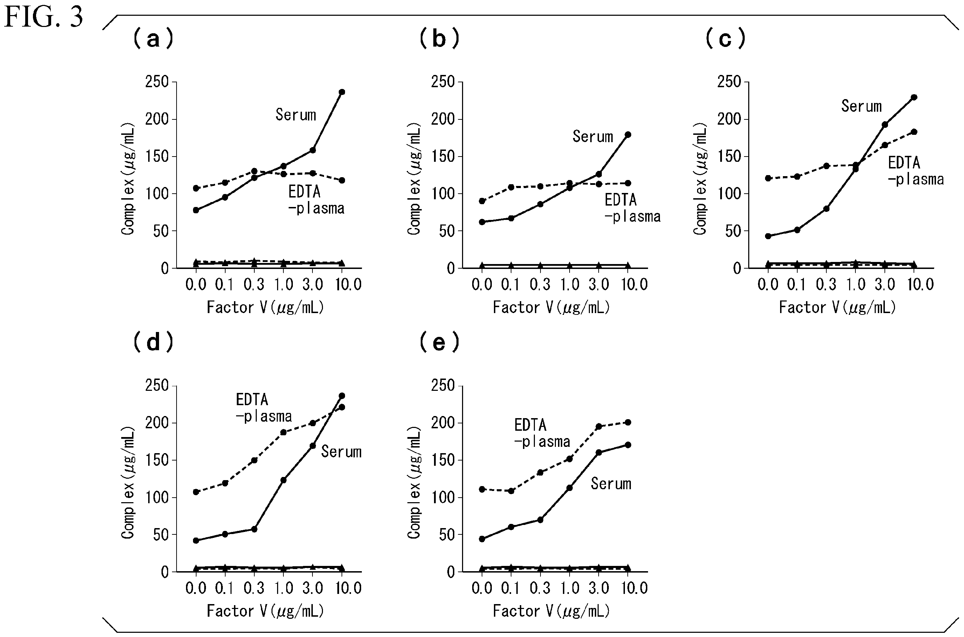

FIG. 3 shows the results of detection by the ELISA method. FIG. 3 (a), (b), (c), (d), and (e) show the results when using plasma and serum samples collected from different subjects (A, B, C, D, E), respectively. The "Complex" on the vertical axis indicates the concentration of the anti-apoA1 antibody detected against the modified HDL forming a complex with the immobilized LOX-1 (including those interposed with other proteins in the binding between LOX-1 and the modified HDL), and the horizontal axis indicates the concentration of Factor V added.

With regard to the serum, as shown in FIGS. 3(a), (b), (c), (d) and (e), in all five samples collected from the subjects A, B, C, D, and E, the detection sensitivity of modified HDL was improved depending on the amount of Factor V added. With regard to the plasma, depending on the samples collected, there were cases in which there was no significant difference due to the addition amount, and cases in which the increase was almost dependent on the addition amount.

From these results, it can be considered that the binding between LOX-1 and the modified HDL was interposed with the Factor V added to the serum, which contains less substances than plasma, since serum contains almost no proteins interposed in the binding between LOX-1 and the modified HDL, such as Factor V, etc. On the other hand, plasma sometimes contains proteins interposed in the binding between LOX-1 and the modified HDL, such as Factor V, etc., but the content varies depending on the sample, collection method, storage method, etc. Therefore, it is considered that the effect of the addition of Factor V varies.

These results indicate that Factor V binds to the modified HDL, and the binding between the modified HDL and LOX-1 is formed via Factor V, and the detection sensitivity of the complex containing this binding is improved depending on the addition amount of Factor V.

### (Test Example 2)

By investigating the detection sensitivity with or without immobilization of LOX-1 on a plate, it was investigated whether or not the improvement of detection sensitivity due to Factor V occurred for the complex of LOX-1, Factor V and the modified HDL.

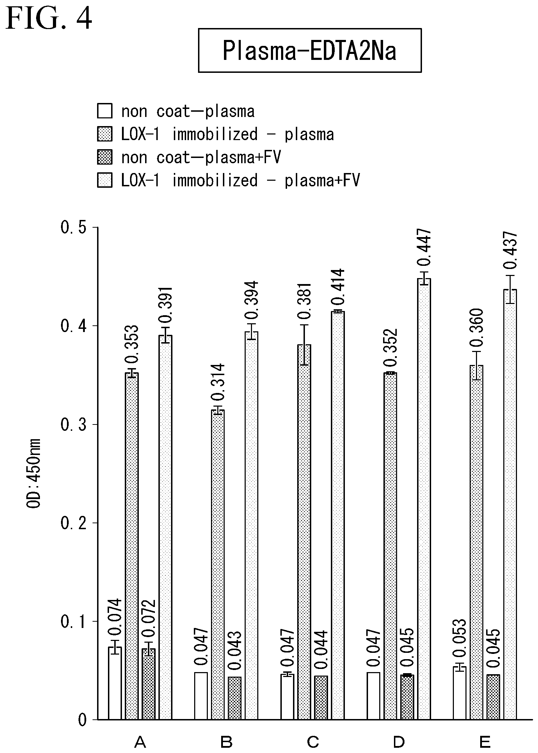

FIG. 4 shows the results of detection by the ELISA method in the same manner as in Test Example 1, except that a plate in which LOX-1 was immobilized (LOX-1 immobilized) and a plate in which LOX-1 was not immobilized (non-coated) were used. In addition, in the detection, plasma samples collected from the subjects A, B, C, D and E were used as the sample, and the detection was carried out in a case of adding a Factor V protein at a concentration of 1 µg/mL (plasma+FV), and a case of not adding Factor V protein (plasma).

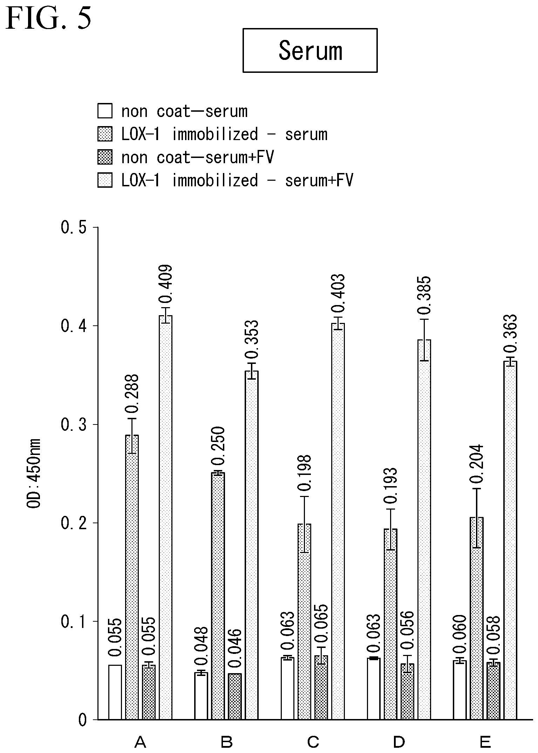

FIG. 5 shows the results of detection by the same method as shown in FIG. 4, except that serum samples collected from the subjects A, B, C, D and E were used as the sample. In addition, the detection was carried out in a case of adding Factor V protein at a concentration of 1 µg/mL (serum+FV), and a case of not adding Factor V protein (serum).

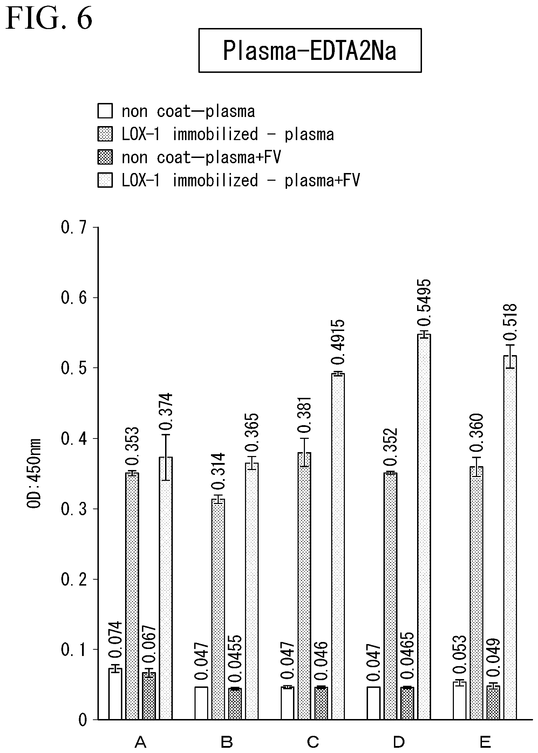

FIG. 6 shows the results of detection by the same method as shown in FIG. 4, except that plasma samples collected from the subjects A, B, C, D and E were used as the sample. In addition, the detection was carried out in a case of adding Factor V protein at a concentration of 10 µg/mL, and a case of not adding Factor V protein.

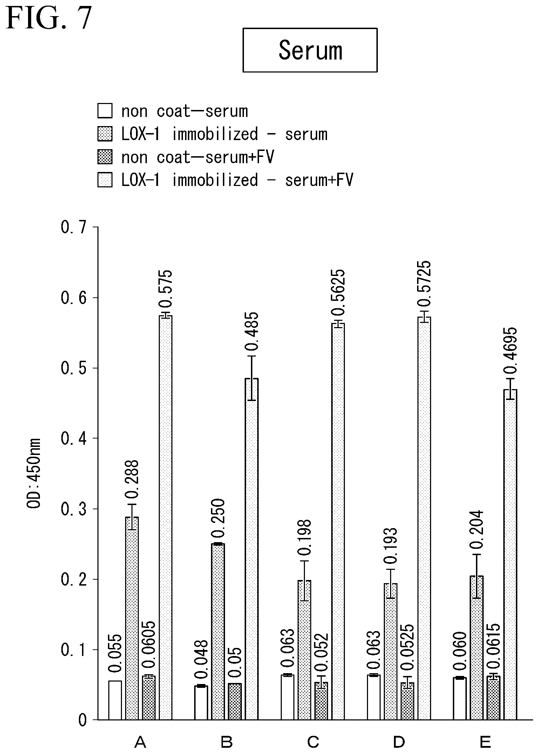

FIG. 7 shows the results of detection by the same method as shown in FIG. 5, except that serum samples collected from the subjects A, B, C, D and E were used as the sample. In addition, the detection was carried out in a case of adding Factor V protein at a concentration of 10 µg/mL, and a case of not adding Factor V protein.

As shown in FIGs. 4 to 7, when a plate on which LOX-1 was not immobilized was used, there was no difference in the detected amount regardless of whether or not Factor V was added to the samples. This result indicates that Factor V improves the detection amount of the complex of LOX-1, Factor V and the modified HDL.

Comparing FIG. 4 with FIG. 5 and comparing FIG. 6 with FIG. 7, it was shown that the detection amount was increased by adding Factor V in FIGs. 5 and 7, in which serum was used, as compared to FIGs. 4 and 6. As with Test Example 1, this result also suggests that the addition of Factor V compensates for the substance that is considered not to be contained in serum.

### (Test Example 3)

Since Factor V binds to the modified HDL, it was attempted to immobilize Factor V directly on the plate to quantify the modified HDL.

First, Factor V dissolved in PBS (-) to a concentration of 0 to 10 µg/mL was added to a plate, and an attempt was made to see if it could be immobilized on a plate by an ELISA method using an anti-Factor V antibody. The conditions were as follows.
Immobilization: Human Factor V at each concentration
Blocking: In 1% casein-Na, 2 hours at room temperature

### Detection:

Primary antibody: Anti-FV-LC (AHV-5101), 1 µg /ml, 1 hour at room temperature
Secondary antibody: Anti-mouse IgG-HRP, 1 :4000, in 1% casein-Na, 1 hour at room temperature

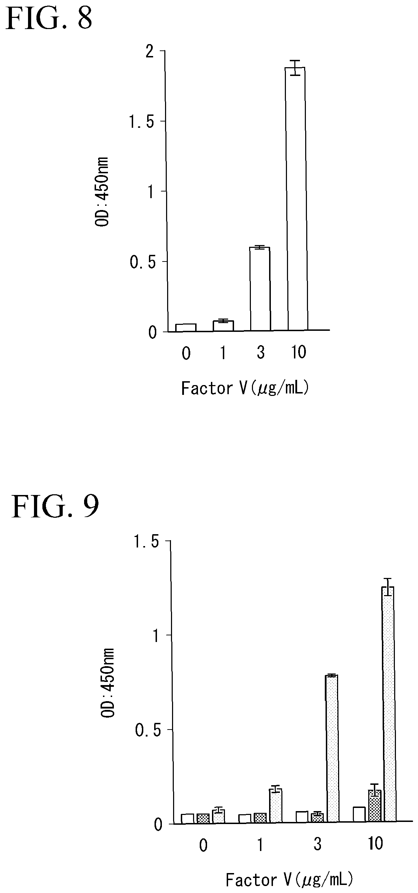

The results are shown in FIG. 8. It was shown that Factor V can be immobilized on the plate almost concentration-dependently.

Subsequently, binding between a non-modified HDL (nHDL) and a modified HDL was tested for this Factor V-immobilized ELISA plate. As the modified HDL, Cu²⁺-oxHDL, which is an oxidized HDL obtained by oxidizing HDL with copper sulfate, was used.

Factor V was immobilized on a 384-well plate (manufactured by Greiner). After washing with PBS twice, blocking was performed with 1 % casein-Na. After washing with PBS three times, the nHDL or the Cu²⁺-oxHDL adjusted to 1 µg/ml with a sample diluent (1% casein-Na, 10 mM HEPES, 150 mM NaCl, pH 7.4, 100 µM APMSF) was incubated at room temperature for 2 hours. After washing with PBS three times, an anti-apoA1 antibody (chicken #1) adjusted to 1 µg/ml was added and incubated at room temperature for 1 hour. After washing with PBS three times, a 4,000-fold diluted HRP-labeled anti-chicken IgY antibody was added and incubated at room temperature for 1 hour. After the antibody reaction, the plate was washed with PBS five times, a TMB solution (manufactured by Bio-Rad) was added to the plate, and the reaction was allowed to proceed at room temperature. The reaction was stopped with 2M sulfuric acid and the absorbance was measured at 450 nm.

The results are shown in FIG. 9. The detection amount of the modified HDL increased depending on the immobilized amount of Factor V on the plate. On the other hand, the detection amount of the nHDL did not change significantly. This result indicates that this plate can be used for detection of modified HDL.

### (Test Example 4)

The modified HDL in human plasma and serum was detected using a Factor V-immobilized plate prepared in the same manner as in Test Example 3. Plasma and serum samples collected from the subjects X and Y were used to quantify the modified HDL by an ELISA method.

Factor V was immobilized on a 384-well plate (manufactured by Greiner). After washing with PBS twice, blocking was performed with 1% casein-Na. After washing with PBS three times, the plasma or serum samples were incubated for 2 hours at room temperature. After washing with PBS three times, an anti-apoA1 antibody (chicken #1) adjusted to 1 µg/ml was added and incubated at room temperature for 1 hour. After washing with PBS three times, a 4,000-fold diluted HRP-labeled anti-chicken IgY antibody was added and incubated at room temperature for 1 hour. After the antibody reaction, the plate was washed with PBS five times, a TMB solution (manufactured by Bio-Rad) was added to the plate, and the reaction was allowed to proceed at room temperature. The reaction was stopped with 2M sulfuric acid and the absorbance was measured at 450 nm.

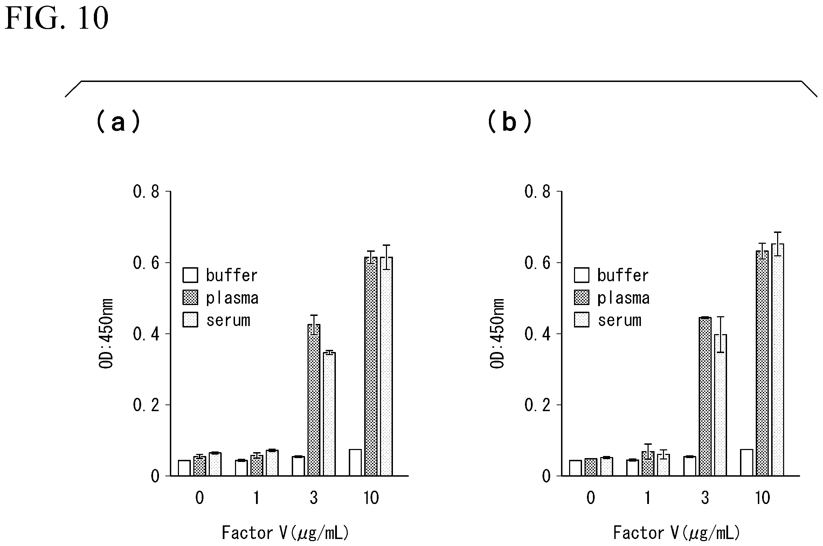

The results are shown in FIG. 10. The results for the sample collected from the subject X is shown in FIG. 10 (a), and the results for the sample collected from the subject Y is shown in FIG. 10 (b).

Unlike the case of immobilizing LOX-1 in the prior art, there was almost no difference in the measured values between plasma and serum. Therefore, it was found that this technique is more sensitive than the technique in which LOX-1 is immobilized, and that this technique not only enables easier quantification than the sandwich method using LOX-1 and Factor V, but also enables stable and reliable quantification with high sensitivity for both plasma and serum.

Although several embodiments of the invention have been described above, these embodiments are presented by way of examples and are not intended to limit the scope of the invention. These embodiments can be implemented in various other forms, and various omissions, replacements, and modifications can be made without departing from the scope of the invention. These embodiments and the modifications thereof are included in the scope and spirit of the invention, as well as the scope of the claimed invention and the equivalents thereof.

### [Industrial Applicability]

According to the present invention, a method for quantifying modified HDL that can quantify modified HDL more stably and accurately than the conventional methods, and an analytical reagent for use in the quantification method, can be obtained. This quantification method and analytical reagent can be used, in particular, to determine the risk or progression of cardiovascular disease, diabetes, and diabetes.

### [Reference Signs List]

10 Modified HDL
20 Modified HDL-binding protein
21 L chain region
22 H chain region
30 Antibody
40, 40A Plate
50 LOX-1 protein

## Claims

1. A method for quantifying modified HDL contained in a sample, comprising:
binding a modified HDL to a modified HDL-binding protein, and
quantifying a complex of the modified HDL-binding protein and the modified HDL, wherein
the modified HDL-binding protein comprises a protein having a sequence of an L chain region of Factor V, or a variant thereof.

2. The method for quantifying modified HDL according to claim 1, wherein the modified HDL-binding protein comprises a sequence of a phospholipid recognition site of Factor V.

3. The method for quantifying modified HDL according to claim 1 or 2, wherein the modified HDL-binding protein comprises a sequence of Factor V.

4. The method for quantifying modified HDL according to any one of claims 1 to 3, wherein the modified HDL-binding protein is a protein having a site partially homologous to a C1-C2 domain, which is a phospholipid recognition site of Factor V.

5. The method for quantifying modified HDL according to claim 4, wherein the protein having a site partially homologous to a C1-C2 domain is MFG-E8, Del-1 or Factor VIII.

6. The method for quantifying modified HDL according to any one of claims 1 to 5, wherein the modified HDL-binding protein is a recombinant protein.

7. The method for quantifying modified HDL according to any one of claim 1 to 6, wherein the quantification is performed by forming a complex of the modified HDL and the modified HDL-binding protein, and quantifying the complex by immunoassay.

8. The method for quantifying modified HDL according to claim 7, wherein the quantification by immunoassay is performed by an ELISA method using an ELISA plate on which the modified HDL-binding protein or a protein that binds to the modified HDL-binding protein is immobilized.

9. The method for quantifying modified HDL according to any one of claims 1 to 8, wherein the quantification is performed by forming a complex of the modified HDL, the modified HDL-binding protein and an LOX-1 protein, and quantifying the complex by immunoassay.

10. The method for quantifying modified HDL according to any one of claims 1 to 9, wherein serum of plasma is used as a sample in the quantification.

11. The method for quantifying modified HDL according to any one of claims 1 to 10, further comprising
adding the modified HDL-binding protein to a sample, wherein
serum is used as a sample in the quantification.

12. An analytical reagent used for quantifying modified HDL for determining cardiovascular disease, diabetes or diabetic disease, or the risk thereof, or the degree of progression thereof, comprising
a modified HDL-binding protein capable of binding to a modified HDL, wherein
the modified HDL-binding protein comprises a protein having a sequence of an L chain region of Factor V, of a variant thereof.

13. The analytical reagent according to claim 12, wherein the modified HDL-binding protein comprises a sequence of a phospholipid recognition site of Factor V.

14. The analytical reagent according to claim 12 or 13, wherein the modified HDL-binding protein comprises a sequence of Factor V.

15. The analytical reagent according to any one of claims 12 to 14, wherein the modified HDL-binding protein is a protein having a site partially homologous to a C1-C2 domain, which is a phospholipid recognition site of Factor V.

16. The analytical reagent according to claim 15, wherein the protein having a site partially homologous to a C1-C2 domain is MFG-E8, Del-1 or Factor VIII.

17. The analytical reagent according to any one of claims 12 to 16, wherein the modified HDL-binding protein is a recombinant protein.

18. The analytical reagent according to any one of claims 12 to 17, wherein the cardiovascular disease is arteriosclerosis, or myocardial infarction or stroke caused by a progression of arteriosclerosis.
